(19) Europäisches Patentamt
European Patent Office
Office européen des brevets

(11) Publication number: **0 405 151 A1**

(12) **EUROPEAN PATENT APPLICATION**

(21) Application number: **90110116.2**

(22) Date of filing: **28.05.90**

(51) Int. Cl.[5]: **C12P 17/02**, C12P 17/18, C07D 498/02, A61K 31/335, A61K 31/42, C12P 1/06, A23K 1/17, //(C12P1/06, C12R1:29)

(30) Priority: **29.06.89 US 373357**
**30.11.89 US 444077**

(43) Date of publication of application:
**02.01.91 Bulletin 91/01**

(84) Designated Contracting States:
**AT BE CH DE DK ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Conner, Susan Diane**
**1009 Hughes Drive, Apt. 14**
**Hamilton Square, New Jersey 08690(US)**
Inventor: **Carter, Guy Thomas**
**8 Prairie Avenue**
**Suffern, New York 10901(US)**
Inventor: **Goodman, Joseph Jacob**
**134 Grotke Road**
**Spring Valley, New York 10977(US)**
Inventor: **Labeda, David Paul**
**2320 West Pintura Court**
**Peoria, Illinois 61614(US)**
Inventor: **Buckwalter, Brian Lee**
**102 Ovington Road**
**Yardley Pennsylvania 19067(US)**

(74) Representative: **Wächtershäuser, Günter, Dr.**
**Tal 29**
**D-8000 München 2(DE)**

(54) **Antibiotic LL-E19085.**

(57) This disclosure describes novel antibacterial agents designated LL-E19085$\beta$, LL-E19085$\gamma$, LL-E19085$\zeta$ and LL-E19085$\eta$, their production by aerobic fermentation of a new subspecies of Micromonospora citrea NRRL 18351 and mutants thereof, and the isolation and purification thereof.

This invention provides methods for increasing the growth rate of warm-blooded animals, for improving the efficiency of feed utilization by said animals, for increasing lactation in lactating ruminant animals and for increasing the wool production of sheep. The invention also relates to novel animal feed compositions and parenteral compositions useful in obtaining the above-described results.

EP 0 405 151 A1

# ANTIBIOTIC LL-E19085

## SUMMARY OF THE INVENTION

This invention relates to methods and compositions for increasing the growth rate of warm-blooded animals, improving the efficiency of feed utilization thereby, enhancing lactation in lactating ruminant animals and increasing wool production in sheep. It also relates to animal feed compositions comprising an edible feed stuff and a sufficient amount of antibiotic LL-E19085η or physiologically acceptable salts thereof to increase the growth rate, improve the efficiency of feed utilization of warm-blooded animals, enhance lactation in lactating ruminants and increase wool production in sheep.

This invention relates to new antibacterial agents designated LL-E19085α, LL-E19085β, LL-E19085γ, LL-E19085ζ and LL-E19085η, to their production by fermentation, to methods for their recovery and concentration from crude solutions and to processes for their purification. The present invention includes within its scope the antibacterial agents in dilute form, as crude concentrates and in pure form. The effect of these new agents on specific microorganisms, together with their chemical and physical properties, differentiate them from previously described antibacterial agents. While not yet fully elucidated, the structure of antibiotic LL-E19085 is proposed as follows:

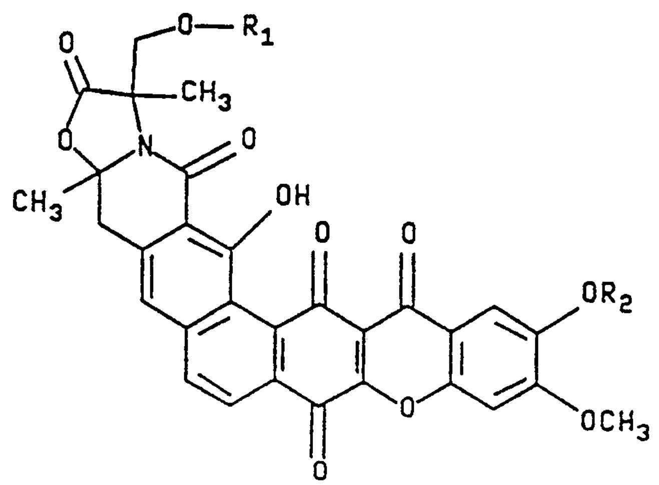

| E19085 | R1 | R2 |
|---|---|---|
| α | $COCH_2CH(CH_3)_2$ | $CH_3$ |
| β | $COCH(CH_3)_2$ | $CH_3$ |
| γ | $COCH_3$ | $CH_3$ |
| ζ | $COCH_2CH(CH_3)_2$ | H |
| η | H | $CH_3$ |

The physiochemical characteristics of antibiotic LL-E19085α are as follows:

a) Molecular formula: $C_{36}H_{31}NO_{12}$;

b) Molecular weight: 669 FABMS $(M+3H)^+$ = M/Z 672.21176;

c) Elemental Analysis: C, 64.55; H, 4.46; N, 1.92;

d) Specific rotation: $[\alpha]_D^{26}$ = -52° (C, 1.11% - di-chloromethane);

e) Ultraviolet absorption spectra:

λmax nm ε 0.1N HCl = 224nm (27,700), 255nm (21,900), 328nm (16,400), 420nm (4,230)

λmax nm ε 0.1N NaOH = 217nm (76,100), 340nm (15,100), 399nm (12,900)

λmax nm ε $CH_3OH$ = 222nm (38,200), 240nm (30,100), 255nm (30,100), 321nm (24,500), 384nm (8,050)

f) Infrared absorption spectrum: (KBr disk), max ($cm^{-1}$) 1800, 1742, 1690, 1621, 1422, 1273;

g) Proton magnetic resonance spectrum: ($CDCl_3$) shows significant peaks as follows:

| δ | #H | M | J(Hz) |
|---|---|---|---|
| 13.47 | 1 | s | - |
| 8.20 | 1 | d | 8.5 |
| 7.92 | 1 | d | 8.5 |
| 7.58 | 1 | s | - |
| 7.19 | 1 | s | - |
| 7.09 | 1 | s | - |
| 4.78 | 1 | d | 11.6 |
| 4.45 | 1 | d | 11.6 |
| 4.02 | 3 | s | - |
| 3.99 | 3 | s | - |
| 3.48 | 1 | d | 12.8 |
| 3.35 | 1 | d | 12.8 |
| 2.21 | 2 | d | 6.8 |
| 2.10 | 1 | m | - |
| 1.83 | 3 | s | - |
| 1.80 | 3 | s | - |
| 0.955 | 6 | d | 6.48 |

h) Carbon-13 nuclear magentic resonance spectrum: ($CDCl_3$) shows significant peaks as follows:

| δ | M | δ | M | δ | M |
|---|---|---|---|---|---|
| 181.2 | s | 134.8 | s | 65.0 | t |
| 178.1 | s | 132.1 | d | 62.3 | s |
| 172.1* | s | 129.7 | s | 56.8 | q |
| 171.5 | s | 124.4 | d | 56.5 | q |
| 165.8 | s | 120.8 | s | 42.9 | t |
| 162.1 | s | 119.9 | s | 41.9 | t |
| 155.5 | s | 119.5 | s | 25.8 | q |
| 153.4 | s | 117.7 | d | 25.5 | d |
| 150.8 | s | 107.3 | s | 22.4 | q |
| 148.7 | s | 104.9 | d | 22.3 | q |
| 140.7 | s | 100.4 | d | 20.2 | q |
| 137.7 | s | 93.4 | s | | |

*Two superimposed resonances.

The physiochemical characteristics of antibiotic LL-E19085β are as follows:

a) Molecular formula: $C_{35}H_{29}NO_{12}$;

b) Molecular weight: 655;

c) ultraviolet absorption spectra: λmax ($CH_3CN$/0.05 M pH 4.5 $NH_4OAC$ 3/2) 258nm, 325nm, 385nm;

d) Infrared absorption spectra: λmax (cm$^{-1}$] 3434br, 2924, 1804, 1752, 1693, 1621, 1426, 1272;

e) Proton nuclear magnetic resonance spectrum: $CDCl_3$ shows significant peaks as follows:

| δ | #H | M | J(Hz) |
|---|---|---|---|
| 13.50 | 1 | br | - |
| 8.26 | 1 | d | 8.5 |
| 7.94 | 1 | d | 8.5 |
| 7.66 | 1 | s | - |
| 7.21 | 1 | s | - |
| 7.14 | 1 | s | - |
| 4.77 | 1 | d | 11.6 |
| 4.47 | 1 | d | 11.6 |
| 4.04 | 3 | s | - |
| 4.02 | 3 | s | - |
| 3.50 | 1 | d | 14.8 |
| 3.36 | 1 | d | 14.8 |
| 2.55 | 1 | septet | 7.0 |
| 1.83 | 3 | s | - |
| 1.77 | 3 | s | - |
| 1.19 | 3 | d | 7.0 |
| 1.18 | 3 | d | 7.0 |

f) HPLC * retention time: 2.4 minutes.

The physiochemical characteristics of antibiotic LL-E19085γ are as follows:

a) Molecular formula: $C_{33}H_{25}NO_{12}$;

b) Molecular weight: 627

c) Ultraviolet absorption spectra: λmax ($CH_3CN$/0.05 M pH 4.5 $NH_4OAC$ 3/2) 258nm, 325nm, 385nm;

d) Infrared absorption spectra: λmax (KBr) $cm^{-1}$: 3433br, 2925, 1805, 1745, 1694, 1621, 1426, 1272;

e) Proton nuclear magnetic resonance spectrum: $CDCl_3$ shows significant peaks as follows:

| δ | #H | M | J(Hz) |
|---|---|---|---|
| 13.46 | 1 | br | - |
| 8.26 | 1 | d | 8.6 |
| 7.94 | 1 | d | 8.6 |
| 7.66 | 1 | s | - |
| 7.21 | 1 | s | - |
| 7.14 | 1 | s | - |
| 4.81 | 1 | d | 11.5 |
| 4.02 | 3 | s | - |
| 3.50 | 1 | d | 14.9 |
| 3.36 | 1 | d | 14.9 |
| 2.10 | 3 | s | - |
| 1.82 | 3 | s | - |
| 1.75 | 3 | s | - |

f) HPLC * retention time: 1.6 minutes.

The physiochemical charactistics of antibiotic LL-E19085ζ are as follows:

a) Molecular formula: $C_{35}H_{29}NO_{12}$;

b) Molecular weight: 655;

* Analytical hplc system. The system consisted of a $C_{18}$ reversed-phase column (2.1 mm X 100 mm) eluted with 60% ACN 40% 0.05 M pH 4.5 $NH_4OAc$ at 0.5 ml/min. Detection was by absorbance at 325 nm.

* Analytical hplc system. Same conditions as used in LL-E19085β recited above.

c) Ultraviolet absorption spectra: λmax ($CH3CN/0.05$ M pH 4.5 $NH_4OAC$ 3/2): 258nm, 324nm, 380nm, 420nm;
d) Infrared absorption spectrum: (KBr) λmax ($Cm^{-1}$) 3429(br), 2960, 1805, 1745, 1693, 1626, 1421, 1279;
e) Proton nuclear magnetic resonance spectrum: $CDCl_3$

| δ | #H | M | J(Hz) |
|---|---|---|---|
| 13.45 | 1 | s | - |
| 8.22 | 1 | d | 8.5 |
| 7.91 | 1 | d | 8.5 |
| 7.75 | 1 | s | - |
| 7.26 | 1 | s | - |
| 7.14 | 1 | s | - |
| 6.20 | 1 | br | - |
| 4.76 | 1 | d | 11.5 |
| 4.45 | 1 | d | 11.5 |
| 4.06 | 3 | s | - |
| 3.48 | 1 | d | 14.8 |
| 3.35 | 1 | d | 14.8 |
| 2.20 | 2 | d | 6.6 |
| 2.10 | 1 | septet | 6.6 |
| 1.81 | 3 | s | - |
| 1.75 | 3 | s | - |
| .950 | 6 | d | 6.6 |

f) Carbon 13 nuclear magnetic resonance spectra:

| δ | δ |
|---|---|
| 181.4 | 119.7 |
| 178.0 | 119.6 |
| 172.8 | 117.7 |
| 171.7 | 108.4 |
| 171.6 | 107.1 |
| 165.6 | 100.2 |
| 161.6 | 93.4 |
| 154.7 | 64.9 |
| 153.5 | 62.3 |
| 150.2 | 56.5 |
| 146.3 | 42.8 |
| 140.5 | 41.7 |
| 137.5 | 25.6 |
| 134.6 | 25.4 |
| 132.2 | 22.2* |
| 129.7 | 20.0 |
| 124.2 | |
| 120.1 | |

* two superimposed signals

g) HPLC * retention time: 1.9 minutes

The physiochemical characteristics of antibiotic LL-E19085η are as follows:

a) Molecular formula: $C_{31}H_{23}NO_{11}$;

* Analytical hplc system. Same conditions as in LL-E19085β recited above.

b) Molecular weight: 585 HRFABMS: $(M + 3H)^+$ = M/Z 588.1479;
c) Specific rotation: $[\alpha]_D^{26\,0}$ = -63° (C .19)DMSO;
d) Ultra violet absorption spectrum: λmax ($MeOH/CH_2CL_2$ 1:1) 223 (37,800), 321 (21,700), 302 (21,750);
e) Infrared absorption spectrum: (KBr) λmax $CM^{-1}$ 3437(br), 2923, 1798, 1691, 1623, 1427, 1276;
f) Proton nuclear magentic resonance spectrum: ($CDCl_3$/DMSO $d_6$)

| δ | #H | M | J(Hz) |
|---|---|---|---|
| 8.24 | 1 | d | 8.5 |
| 7.96 | 1 | d | 8.5 |
| 7.63 | 1 | s | - |
| 7.23 | 1 | s | - |
| 7.17 | 1 | s | - |
| 5.28 | 1 | br | - |
| 4.33 | 1 | brd | 10.1 |
| 4.04 | 3 | s | - |
| 4.02 | 3 | s | - |
| 3.90 | 1 | brd | 10.1 |
| 3.51 | 1 | d | 14.7 |
| 3.37 | 1 | d | 14.7 |
| 1.77 | 3 | s | - |
| 1.71 | 3 | s | - |

g) Carbon 13 nuclear magnetic resonance spectrum: (DMSO $D_6$/$CDCl_3$)

| δ | δ |
|---|---|
| 180.9 | 107.4 |
| 177.7 | 104.4 |
| 173.0 | 100.7 |
| 171.9 | 93.3 |
| 165.8 | 65.8 |
| 161.4 | 63.4 |
| 155.6 | 56.7 |
| 153.8 | 56.1 |
| 150.8 | 41.4 |
| 148.6 | 25.3 |
| 140.3 | 19.0 |
| 137.3 | |
| 135.2 | |
| 132.2 | |
| 129.6 | |
| 123.9 | |
| 120.2 | |
| 119.3 | |
| 119.2 | |
| 117.6 | |

h) HPLC * retention time: 1.2 minutes

## DETAILED DESCRIPTION OF THE INVENTION

* Analytical hplc system . Same conditions as in LL-E19085β recited above.

The antibacterial agents LL-E19085α, LL-E19085β, LL-E19085γ, LL-E19085ζ and LL-E19085η are produced by aerobic fermentation of microbial culture LL-E19085 which is a natural selection isolate of a culture isolated from a soil sample collected in Manyara, Tanzania. The culture was taxonomically characterized and identified as a new subspecies of *Micromonospora citrea*.

This new subspecies is maintained in the culture collection of the Medical Research Division, American Cyanamid Company, Pearl River, NY as culture number LL-E19085. A viable culture of this new microorganism has been deposited under the terms of the Budapest Treaty with the ARS Culture Collection, Fermentation Laboratory, Northern Regional Research Center, U. S. Department of Agriculture, 1815 North University Street, Peoria, IL 61604 and has been added to its permanent collection. It has been assigned the strain designation NRRL 18351 by such depository.

Observations were made of the cultural, physiological and morphological features of culture LL-E19085 using methods well known in the art. The generic assignment of LL-E19085 to the genus *Micromonospora* was confirmed morphologically and chemically. The strain produced monospores on the vegetative hyphae. No aerial hyphae were observed. Electron microscopic examination showed that the spores were warty. Whole cell analysis showed that LL-E19085 contained the *meso* isomer as well as traces of the L isomer of diaminopimelic acid. This strain showed the presence of xylose plus traces of arabinose in its whole cell sugar hydrolysates. Therefore LL-E19085 is considered to be a subspecies of *Micromonospora citrea*.

Comparative date on the morphology of LL-E19085 is given in Tables I and II. Physiological data is given in Tables III and IV.

TABLE I

| ISP Agar Medium | Spores | Vegetative Mycelium | Soluble Pigments |
|---|---|---|---|
| Yeast-Malt (ISP 2) | Slight, black at edge | Strong orange (50) to medium orange-yellow (71*) | Slight, brown-black |
| Oatmeal (ISP 3) | None | Light orange-yellow (70) to vivid orange-yellow (66) | Slight, brownish |
| Inorganic Salts-Starch (ISP 4) | None | Light orange-yellow (70) to vivid orange-yellow (66) | Slight, brownish |
| Glycerol-Asparagine (ISP 5) | Slight, brownish at edge | Light tone of brownish-orange (54) | Slight, brownish |

*Parenthetical numbers are colors taken from Kelly, K.L. and Judd, D. B., Color. Universal Language and Dictionary of Names, Nat. Bur. Stand. (U. S.), Spec. Publ. 440, 1976, Washington, D. C. and the accompanying Inter-Society Colo Council, National Bureau of Standards Centroid Color Charts.

TABLE II

| Agar Medium | Actinomycete Growth (28°C, 2 weeks) |
|---|---|
| Pablum | Brown vegetative hyphae. Sparse spores. Soluble dark brown pigment. |
| Yeast Czapek's | Brownish tan vegetative hyphae. Sparse spores. Slight soluble dark-brownish pigment. |
| Czapek's | Vegetative hyphae covered/spores. Black spores. Slight dark pigment. |
| Yeast Extract-Dextrose | Black spores, Dry soluble brownish pigment. |
| Nutrient | Orange-brown vegetative hyphae. Sparse black spores. Moderate brown pigment. |
| Nutrient Glycerol | Blackish-tan vegetative hyphae. Sparse spores. Intense brown-black pigment. |
| Bennett's Dextrin | Tan vegetative hyphae. Moderate black spores. Soluble reddish-brown pigment. |
| Glucose Asparagine | Orange-tan vegetative hyphae. No spores. Slight soluble dark pigment. |

TABLE III

| Carbohydrate | Carbohydrate Utilization |
|---|---|
| Arabinose | + |
| Cellulose | - |
| Fructose | ± |
| Glucose | + |
| Inositol | - |
| Mannitol | - |
| Raffinose | - |
| Rhamnose | - |
| Sucrose | ± |
| Xylose | + |

TABLE IV

| Gordon Test | Physiological Reaction |
|---|---|
| **Hydrolysis of** | |
| Casein | + |
| Xanthine | - |
| Hypoxanthine | - |
| Tyrosine | + |
| Adenine | + |
| Gelatin | + |
| Potato Starch | + |
| Esculin | + |
| Physiological | |
| **Production of** | |
| Nitrate Reductase | - |
| Phosphatase | + |
| Urease | - |
| **Growth on** | |
| Salicin | - |
| 5% Sodium Chloride | - |
| Lysozyme Broth | - |
| **Decarboxylation of** | |
| Acetate | + |
| Benzoate | - |
| Citrate | - |
| Lactate | + |
| Malate | - |
| Mucate | - |
| Oxalate | - |
| Propionate | + |
| Pyruvate | + |
| Succinate | - |
| Tartrate | - |

TABLE IV (continued)

| Gordon Test | Physiological Reaction |
|---|---|
| **Acid from** | |
| Adonitol | - |
| Arabinose | + |
| Cellobiose | + |
| Dextrin | + |
| Dulcitol | - |
| Erythritol | - |
| Fructose | + |
| Galactose | + |
| Glucose | + |
| Glycerol | + |
| Inositol | - |
| Lactose | + |
| Maltose | + |
| Mannitol | - |
| Mannose | + |
| α-Methyl-D-glucoside | + |
| Melibiose | + |
| Raffinose | + |
| Rhamnose | - |
| Salicin | + |
| Sorbitol | - |
| Sucrose | + |
| Trehalose | + |
| Xylose | + |
| β-Methyl-D-xyloside | - |
| **Growth at** | |
| 10°C | - |
| 42°C | + |
| 45°C | + |

+ = positive; - = negative

It is to be understood that for the production of the new antibacterial agents LL-E19085α, LL-E19085β, LL-E19085γ, LL-E19085ζ and LL-E19085η, the present invention is not limited to this particular organism or to organisms fully answering the above growth and microscopic characteristics, which are given for illustrative purposes only. In fact it is desired and intended to include the use of mutants produced from this organism by various means such as exposure to x-radiation, ultraviolet radiation, N′-methyl-N′-nitro-N-nitrosoguanidine, actinophages and the like.

The in vitro antibacterial effects of antibiotics LL-E19085α, LL-E19085β, LL-E19085γ, LL-E19085ζ and LL-E19085η were determined by standard agar dilution methods against clinical isolates obtained from medical centers representing various geographical areas in the United States. The inoculum of each culture was approximately 1 to $5x10^4$ colony forming units applied with a Steers multiple inocula replicator to plates containing the antibiotic in Mueller-Hinton agar. The agar was supplemented with about 5% sheep blood where required for the growth of the organism. The results are given in Table V.

**TABLE V**

**In vitro Antibacterial Activity of LL-E19085α**

| Organism | | Minimal Inhibitory Concentration (mcg/ml) |
|---|---|---|
| *Staphylococcus aureus* | ATCC 25923 | 0.12 |
| *Staphylococcus aureus* | Smith | 0.12 |
| *Staphylococcus aureus* | VGH 84-45 | ≤0.06 |
| *Staphylococcus aureus* | CMC 83-127 | ≤0.06 |

TABLE V (continued)

| Organism | | Minimal Inhibitory Concentration (mcg/ml) |
|---|---|---|
| Staphylococcus aureus | CMC 83-131 | ≤0.06 |
| Staphylococcus aureus | CMC 83-132 | ≤0.06 |
| Staphylococcus aureus | SSC 82-57 | ≤0.06 |
| Staphylococcus epidermidis | IO 83-58 | ≤0.06 |
| Staphylococcus saphrophiticus | VGH 84-50 | ≤0.06 |
| Streptococcus β-hemolyticus | C 203 | ≤0.06 |
| Streptococcus β-hemolyticus | VGH 84-60 | ≤0.06 |
| Streptococcus β-hemolyticus | VGH 84-61 | ≤0.06 |
| Streptococcus β-hemolyticus | VGH 84-62 | ≤0.06 |
| Streptococcus pneumoniae | SV-1 | ≤0.06 |
| Streptococcus pneumoniae | K 84-21 | ≤0.06 |
| Enterococcus | VGH 84-65 | ≤0.06 |
| Enterococcus | VGH 84-68 | ≤0.06 |
| Enterococcus | IO 83-28 | ≤0.06 |
| Enterococcus | IO 83-40 | 0.12 |
| Enterococcus | CMC 83-72 | ≤0.06 |
| Escherichia coli | No. 311 | >128 |
| Klebsiella pneumoniae | AD | >128 |
| Enterobacter cloacae | VGH 84-37 | >128 |
| Morganella morganii | VGH 84-11 | >128 |
| Serratia marcescens | K 84-14 | >128 |
| Pseudomonas aeruginosa | 12-4-4 | >128 |
| Citrobacter diversis | MOR 84-3 | >128 |
| Proteus vulgaris | CMC 84-35 | >128 |
| Alcaligenes ssp. | ISG 86-34 | >128 |
| erratia rubidea | UHL 86-4 | >128 |
| Bacteroides fragilis | ATCC 25285 | 16 |

| Organism | | Minimal Inhibitory Concentration (mcg/ml) |
|---|---|---|
| *Bacteroides vulgaris* | ATCC 29327 | ≤0.06 |
| *Bacteroides theta* | ATCC 29741 | 4 |
| *Bacteroides theta* | ATCC 29742 | 4 |
| *Clostridium perfringen* | ATCC 13124 | ≤0.06 |
| *Clostridium differensis* | ATCC 17858 | ≤0.06 |
| *Peptococcus magnus* | ATCC 29328 | ≤0.06 |
| *Peptococcus magnus* | ATCC 14956 | ≤0.06 |
| *Peptococcus asacrolytis* | ATCC 29743 | ≤0.06 |
| *Escherichia coli* | ATCC 25922 | >128 |
| *Staphylococcus aureus* | ATCC 29213 | ≤0.06 |

TABLE VI

Invitro Antibacterial Activity of LL-E19085β, γ, δ, η

| Organism | | Minimal Inhibitory Concentration (mcg/ml) LL-E19085β | LL-E19085γ | LL-E19085δ | LL-E19085η |
|---|---|---|---|---|---|
| Staphylococcus aureus | NEMC 87-69 | 4 | 4 | 8 | ≤.015 |
| Staphylococcus aureus | ROSE (MP) | 2 | 1 | 2 | ≤.015 |
| Staphylococcus aureus | IVES 542 | 4 | 8 | 8 | ≤.015 |
| Staphylococcus hemolyticus | AVAH 88-1 | 2 | 8 | 4 | ≤.015 |
| Staphylococcus hemolyticus | AVAH 88-3 | 4 | 0.5 | 2 | ≤.015 |
| Bacteroides cereus | DAVIES | 2 | 0.5 | 2 | ≤.015 |
| Streptococcus faecalis | ARUM 87-41 | 2 | 0.5 | 2 | ≤.015 |
| Enterococcus | CHBM 88-60 | 2 | 0.5 | 2 | ≤.015 |
| Enterococcus | Grp D WRVA 88-33 | 2 | 0.5 | 2 | ≤.015 |
| Streptococcus faecalis | VCI 85-30 | 2 | .06 | 2 | ≤.015 |
| Streptococcus faecalis | VCH 84-69 | 2 | .03 | 2 | ≤.015 |
| Streptococcus faecalis | CMC 83-120 | 2 | 1 | 2 | ≤.015 |
| Streptococcus a-hemolyticus | AMCH 88-84 | 0.5 | ≤.008 | 1 | ≤.015 |
| Streptococcus b-hemolyticus | AMCH 88-86 | 2 | ≤.008 | 2 | ≤.015 |
| Streptococcus pneumoniae | CHBM 88-70 | .25 | ≤.008 | .12 | ≤.015 |
| Streptococcus pneumoniae | CHBB 88-75 | .12 | ≤.008 | .12 | ≤.015 |
| Streptococcus pneumoniae | TEX 85-2 | .12 | ≤.008 | .12 | ≤.015 |
| Staphylococcus aureus | ATCC 29213 | 2 | 1 | 2 | ≤.015 |
| Klebsiella pneumoniae | NEMC 87-271 | ≥64 | ≥16 | ≥32 | ≥32 |
| Escherichia coli | D21 | ≥64 | ≥16 | ≥32 | ≥32 |

TABLE VI (continued)

Invitro Antibacterial Activity of LL-E19085β, γ, δ, η

| Organism | | Minimal Inhibitory Concentration (mcg/ml) LL-E19085β | LL-E19085γ | LL-E19085δ | LL-E19085η |
|---|---|---|---|---|---|
| *Escherichia coli* | D22 | 8 | 16 | 32 | 2 |
| *Escherichia coli* | ATCC 25922 | >64 | >16 | >32 | >32 |
| *Escherichia coli* | ATCC 35218 | >64 | >16 | >32 | >32 |
| *Staphylococcus aureus* | ATCC 25923 | 0.5 | ≥0.008 | 1 | ≤0.015 |
| *Staphylococcus aureus* | VGH 84-47 | 4 | 1 | 4 | ≤0.015 |
| *Staphylococcus aureus* | K82-26 | 2 | ≤0.008 | 2 | ≤0.015 |
| *Staphylococcus aureus* | CMC 83-131 | 4 | 16 | 8 | 0.12 |

The antibiotic LL-E19085$\eta$ or salts thereof are useful for the treatment of both monogastric and ruminant animals. Moreover, said antibiotic is particularly effective for improving feed efficiency and inducing weight gains in warm-blooded species such as cattle, sheep, swine, goats, rabbits, horses, cats, dogs, fur-bearing livestock such as mink and fox, poultry such as chickens, ducks, geese, turkeys and zoological specimens.

In accordance with the invention, the antibiotic LL-E19085$\eta$ or salts thereof may be orally or parenterally administered to the animals. It may be administered in admixture with the animal's feed or as a top dressing thereto. It may also be proffered to said animals in the form of a bolus, pellet, tablet, pill, drench, oral gel or the like, or provided in the animal's drinking water.

When orally administered in the feed or drinking water, generally about 0.01 ppm to 1000 ppm of the antibiotic LL-E19085$\eta$ or salts thereof in the feed or drinking water is effective for enhancing the growth rate and improving the efficiency of feed utilization by the host animals. When orally administered in the feed or drinking water, generally about 0.01 ppm to 200 ppm of said antibiotic in the feed or drinking water is effective for increasing lactation in lactating ruminants.

Increased wool production in sheep can be achieved by oral administration of the antibiotic LL-E19085$\eta$ or salts thereof at concentrations of about 0.01 ppm to 1000 ppm in or with the feed or drinking water of said sheep. Wool production may also be increased by parenterally administering to sheep the antibiotic LL-E19085$\eta$ in sufficient amounts to provide the sheep with from about 0.0001 mg/kg of 100 mg/kg of animal body weight per day of said antibiotic.

Since the antibiotics of the present invention are useful in the treatment of both monogastric and ruminant animals which may weigh only a few grams or as much as several thousand kilograms, the effective levels of antibiotic necessary for treating said animals will vary with the animals stage of development and from species to species.

Animal feed compositions which provide the desired growth promotion and feed efficiency in the above-mentioned animals may be prepared by admixing the above said antibiotic or salts thereof, or an animal feed supplement containing said antibiotic, with a sufficient quantity of an appropriate animal feed to provide the desired level of active compound in said feed.

Animal feed supplements may be prepared by admixing about 1.0% to 75% by weight of the antibiotic with about 99% to 25% by weight of a suitable carrier or diluent. Carriers or diluents suitable for use in the preparation of the feed supplements include the following: alfalfa meal, soybean meal, cottonseed oil meal, linseed oil meal, sodium chloride, corn meal, cane molasses, urea, bone meal, fish meal, corncob meal, calcium chloride, and other similar materials. Use of the carriers or diluents in feed supplements promote uniformity of distribution of the active ingredient in the finished feed into which the supplement is blended. It thus performs an important function by ensuring proper distribution of the active ingredient throughout the feed.

If the supplement is used as a top dressing for feed, it helps to ensure uniformity of distribution of the active material across the top of the dressed feed.

For parenteral administration, the antibiotic may be prepared in the form of a paste or pellet and administered as an implant, usually under the skin of the head or ear of the animal in which enhanced growth rate and/or improved efficiency of feed utilization is desired.

In practice, parenteral administration generally involves injection of a sufficient amount of the above said antibiotic to provide the animal with from about 0.0001 to 100 mg/kg of body weight of the active ingredient.

Paste formulations may be prepared by dispersing the antibiotic in a pharmaceutically acceptable oil, such as, for example, peanut oil, sesame oil and corn oil.

Pellets containing an effective level of the antibiotic LL-E19085$\eta$ may be prepared by admixing the above-said antibiotic with a diluent, such as, for example, carbowax, biodegradable polymers, carnauba wax, or the like. A lubricant, such as, for example, magnesium stearate or calcium stearate, may be added to improve the pelleting process if desired.

It is, of course, recognized that more than one pellet may be administered to an animal to achieve the desired dose level which will provide the increased growth rate and/or improve efficiency of feed utilization by said animal. Moreover, it has been found that additional implants may also be introduced periodically during the treatment period in order to maintain the proper drug release rate in the animal's body.

## General Fermentation Conditions

Cultivation of *Micromonospora citrea* sp. LL-E19085 may be carried out in a wide variety of liquid culture media. Media which are useful for the production of antibiotic LL-E19085$\alpha$ include an assimilable

source of carbon, such as starch, sugar, molasses, glycerol, etc.; an assimilable source of nitrogen, such as protein, protein hydrolysate, polypeptides, amino acids, cornsteep liquor, etc.; and inorganic anions and cations, such as potassium, sodium, ammonium, calcium, sulfate, carbonate, phosphate, chloride, etc. Trace elements such as boron, molybdenum, copper, etc., are supplied as impurities of other constituents of the medium. Aeration is supplied by forcing sterile air through or onto the surface of the fermenting medium. Agitation is provided by a mechanical impeller. An antifoam agent may be added as needed. The growth of the organism is usually conducted at about 247°C, preferably at about 28°C.

The following examples describe the invention in detail.

## Example 1

### Inoculum Preparation

A typical medium used to grow the inocula was prepared according to the following formula:

| | |
|---|---|
| Dextrose..... | 1.0% |
| Dextrin..... | 2.0% |
| Yeast extract..... | 0.5% |
| NZ Amine A *..... | 0.5% |
| Calcium carbonate..... | 0.1% |
| Defoam agent..... | 0.3% |
| Water .....qs..... | 100% |

*A pancreatic digest of casein, registered trademark of Sheffield Chemical, Norwich, NY.

This medium was sterilized and a 100ml portion in a 500ml flask was inoculated with mycelial scrapings from an agar slant of the culture *Micromonospora citrea* sp LL-E19085. The inoculated flask was then placed on a rotary shaker and agitated vigorously for approximately 48 hours at 32°C, providing primary inoculum.

A 100ml portion of this primary inoculum was then used to inoculate 10 liters of the above sterile medium which was incubated at 32°C with aeration for 72 hours, providing secondary inoculum.

A 10 liter portion of this secondary inoculum was then used to inoculate 260 liters of the above sterile medium in a tank. This medium was incubated at 32°C with agitation by an impeller driven at 180 rpm, a sterile air flow of 200 liters per minute and the addition of 50 ml of a defoaming agent for about 48 hours, providing tertiary inoculum.

## Example 2

### Fermentation

A fermentation medium was prepared according to the following formulation:

| | |
|---|---|
| Dextrin..... | 3.0% |
| Dextrose..... | 0.5% |
| Nutrisoy..... | 1.5% |
| Corn steep liquor..... | 0.5% |
| Calcium carbonate..... | 0.5% |
| Defoam agent..... | 0.3% |
| Water.....qs..... | 100% |

A 2800 liter portion of the above medium in a tank was sterilized and then inoculated with 300 liters of tertiary inoculum prepared as described in Example 1. Aeration was supplied at the rate of 6.5 liters of sterile air per liter of mash per minute and agitation was supplied by an impeller driven at about 110 rpm. The temperature was maintained at 28°C and defoaming agent was added as required. The fermentation was terminated after 129 hours.

## Example 3

## Isolation of Antibiotic LL-E19085α

A 1500 liter portion of the whole harvest mash, prepared as described in Example 2, was mixed with 15 liters of toluene for 30 minutes, then 250 lb of diatomaceous earth was added. After mixing for 15 minutes this mixture was filtered and the cake washed with 150 liters of water. The cake was slurried in a mixture of 208 liters of acetone, 416 liters of dichloromethane and 20 liters of 1.5N hydrochloric acid for 2 hours and then filtered. The cake was washed with about 175 liters of dichloromethane with the wash and filtrate combined. The cake was then washed with about 800 liters of water and this wash also combined with the above wash and filtrate and mixed. The dichloromethane layer was separated and washed with an equal volume of water. The dichloromethane layer was separated and concentrated to 100 liters, reextracted with fresh methylene chloride if any aqueous phase was present, and finally concentrated to about 1-3 liters.

The dichloromethane extracts were triturated repeatedly, first with hexane:dichloromethane (9:1) and then with hexane alone to remove the bulk of the fatty impurities giving a brown powder.

Several such partially purified preparations, from fermentations conducted as described in Example 2, totaling 20 g and averaging 10-30% LL-E19085α, were combined and purified by reverse-phase chromatography. The column consisted of a 15 liter bed of $C_{18}$ bonded phase packing of 40 micron particle size. The charge was loaded onto the column in 500 ml of acetonitrile:tetrahydrofuran (1:1). The column was developed at a flow rate of 1.0 liter per minute with a mobile phase consisting of acetonitrile:0.1M pH 4.5 ammonium acetate buffer (8:2). Fractions were collected at approximately 12 minute intervals. Fractions 6 and 7 were combined and evaporated, giving 2.7 g of pure LL-E19085α having the characteristics disclosed in the hereinabove specification.

## Example 4

## Isolation of Antibiotics LL-E19085β and LL-E19085γ

These minor components were isolated from various side fractions derived from the refining of LL-E19085a from tank fermentations. Chromatography of the material from methylene chloride ($CH_2Cl_2$), extraction of the whole mash on silica gel, and eluting with mixtures of acetone in $CH_2Cl_2$ yielded a fraction enriched in β and γ. This material was dissolved in acetonitrile (ACN) and separated by reversed phase chromatography, NB 7411C99. The column was 21.4 mm X 25 cm packed with $C_{18}$ bonded silica. The column was eluted with 60% ACN, 40% 0.05 M pH 4.5 $NH_4OAc$ . Fractions were combined on the basis of

analytical hplc to yield $\beta$ and $\gamma$.

Example 5

Isolation of Antibiotic LL-E19085$\eta$

An early fraction from the large-scale $C_{18}$ column used to purify E19085$\alpha$ was used to obtain the $\eta$ component. The material was dissolved in ACN and separated on the 21.4 mm X 25 cm $C_{18}$ column eluting with 50% ACN, 50% 0.05 M pH 4.5 $NH_4OAc$. Fractions were combined on the basis of analytical hplc to yield E19085$\eta$.

Example 6

Isolation of LL-E19085$\zeta$

Side fractions from silica gel purification of E19085$\alpha$ were enriched in E19085$\zeta$ (NB7411C73). The fraction containing zeta was rechromatographed on silica gel (150g Wolen, column 2.5 X 50 cm) eluted with $CH_2Cl_2$ followed by 5% acetone 95% $CH_2Cl_2$. Fractions were combined on the basis of analytical hplc and evaporated to dryness to yield E19085$\zeta$. (7580C151)

**EXAMPLE 7**

**Evaluation of antibiotic LL-E19085$\eta$ for increasing the growth of chickens and improving the efficiency of feed utilization thereby**

In this test one day old Peterson X Arbor Acres chicks are sorted into equal weight groups of 5 males and 5 females per cage. Cages are randomized to treatment groups with six replicates per treatment. Antibiotic LL-E19085$\eta$ is tested at 25 ppm and 50 ppm in the diet and evaluated against chicks receiving a non-medicated diet.

The chicks are weighed at the start of the test and at 1 week intervals thereafter to the conclusion of said test. The chicks are given free access to feed and water during the entire test period. The feed is weighed when provided to the chicks and excess feed collected and weighed when the cages are cleaned.

The poultry diet employed in the test is as follows:

Vitamin-amino acid premix: 0.5%
Trace minerals: 0.1%
Sodium chloride: 0.3%
Dicalcium phosphate: 1.2%
Ground limestone: 0.5%
Stabilized fat: 4.0%
Dehydrated alfalfa, 17% protein: 2.0%
Corn gluten meal,41% protein: 5.0%
Menhaden fish meal, 60% protein: 5.0%
Soybean oil meal, 44% protein: 30.0%
Ground yellow corn, fine to: 100.0%

The vitamin-amino acid premix in the above feed composition is prepared from the following formulation. The expressions of quantity relate to units per kilogram of the finished feed composition.

Butylated hydroxy toluene: 125.0 mg
dl-Methionine: 500.0 mg
Vitamin A: 3300.0 I.U.
Vitamin $D_3$: 1100.0 I.C.U.
Riboflavin: 4.4 mg
Vitamin E: 2.2 I.U.
Niacin: 27.5 mg
Panthothenic acid: 8.8 mg
Choline chloride: 500.0 mg
Folic acid: 1.43 mg
Menadione sodium bisulfate: 1.1 mg
Vitamin $B_{12}$: 11.0 mcg
Ground yellow corn, fine to: 5.0 mg.

Data obtained are reported in Table below where it can be seen that antibiotic LL-E19085η improved the weight gains of chicks and increased the efficiency of feed utilization thereby over unmedicated controls.

TABLE

| Evaluations of Antibiotic LL-E19085η for increasing the growth rate of poultry and improving the efficiency of feed utilization thereby | | | |
|---|---|---|---|
| | | Weight gains % improvement over controls | Feed/gain % improvement over controls |
| Treatment | ppm | 14 Days | 14 Days |
| Control | - | - | - |
| Antibiotic LL-E19085η | 25 | 11.7 | 5.5 |
| Antibiotic LL-E19085η | 50 | 5.0 | 5.5 |

## Claims

1. The compound having the structure:

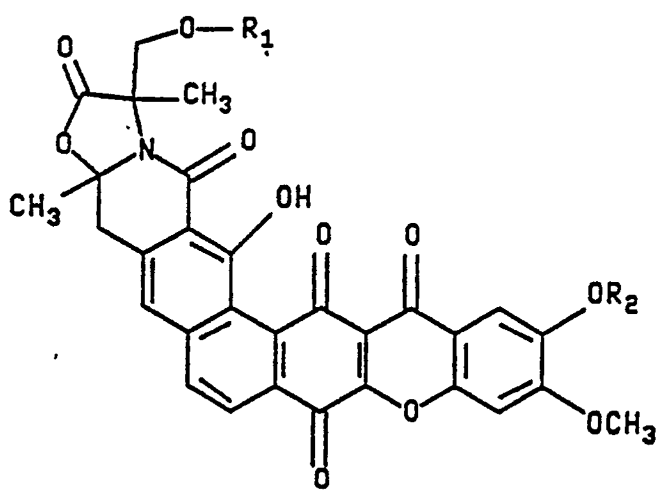

wherein $R_1$ is H, $COCH_3$, or $COCH(CH_3)_2$, and $R_2$ is $CH_3$.

2. The compound having the structure:

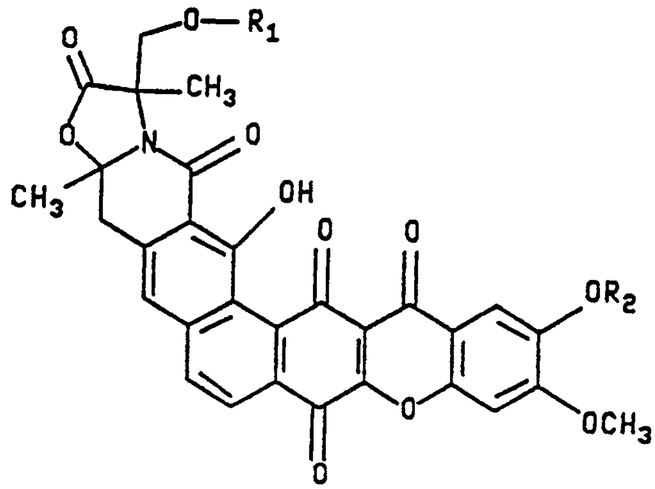

wherein $R_1$ is $COCH_2CH(CH_3)_2$ and $R_2$ is H.

3. A method of treating bacterial infections in warm-blooded animals which comprises administering to said animals an antibacterially effective amount of a compound selected from the group consisting of LL-E19085β, LL-E19085γ, LL-E19085ζ, and LL-E19085η.

4. A process for producing an antibiotic selected from the group consisting of LL-E19085β, LL-E19085γ, LL-E19085ζ, and LL-E19085η which comprises aerobically fermenting the microorganism *Micromonospora citrea* sp. NRRL 18351 or mutants thereof in a liquid medium containing assimilable sources of carbon, nitrogen and inorganic salts, until substantial antibiotic activity is imparted to said medium and then recovering the antibiotic therefrom.

5. The process for producing antibiotics LL-E19085β , LL-E19085γ, LL-E19085ζ and LL-E19085η as recited in Claim 3, which comprises aerobically fermenting a liquid medium containing assimilable sources of carbon, nitrogen and inorganic salts, which medium has been inoculated with a viable culture of the microorganism *Micromonospora citrea* sp. NRRL 18351 or mutants thereof, maintaining said fermentation culture at a temperature of about 24-37°C for a period of 50-150 hours, harvesting the mash and extracting the antibiotic.

6. A method of increasing the growth rate of warm-blooded animals which comprises administering to said animals a growth rate increasing amount of antibiotic LL-E19085η or physiologically acceptable salts thereof.

7. The method according to claim 6, wherein said antibiotic is orally administered to said animals at concentrations of from about 0.01 ppm to 1000 ppm in or with the feed or drinking water of said animals.

8. The method according to claim 6, wherein said antibiotic is parenterally administered to said animals in sufficient amount to provide the animals with from about 0.001 mg/kg to 100 mg/kg of body weight per day of said antibiotic.

9. A method of increasing the efficiency of feed utilization by warm-blooded animals which comprises administering to said animals a feed efficiency increasing amount of antibiotic LL-E19085η or physiologically acceptable salts thereof.

10. An animal feed composition for increasing the growth rate of warm-blooded animals which comprises an edible animal feed and about 0.01 ppm to 1000 ppm of antibiotic LL-E19085η or physiologically acceptable salts thereof.

11. An animal feed composition for increasing the efficiency of food utilization which comprises an edible animal feed and about 0.01 ppm to 1000 ppm of antibiotic LL-E19085η or physiologically acceptable salts thereof.

European Patent Office

# PARTIAL EUROPEAN SEARCH REPORT

which under Rule 45 of the European Patent Convention shall be considered, for the purposes of subsequent proceedings, as the European search report

Application number: EP 90110116.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP - A2 - 0 353 381 (AMERICAN CYANAMID COMPANY) * Page 2; claims 3-7 * -- | 1,2,4, 5 | C 12 P 17/02<br>C 12 P 17/18<br>C 07 D 498/02<br>A 61 K 31/335<br>A 61 K 31/42<br>C 12 P 1/06<br>A 23 K 1/17<br>//(C 12 P 1/06<br>C 12 R 1:29) |
| A | EP - A2 - 0 182 152 (AMERICAN CYANAMID COMPANY) * Claims 18-21 * ---- | 4,5 | |

TECHNICAL FIELDS SEARCHED (Int. Cl.5)

C 12 P
C 07 D
A 61 K
A 23 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1,2,4-11
Claims searched incompletely: –
Claims not searched: 3
Reason for the limitation of the search: Article 52(4) EPC

| Place of search | Date of completion of the search | Examiner |
|---|---|---|
| VIENNA | 12-10-1990 | WOLF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1. 03.82